(19) Europäisches Patentamt
European Patent Office
Office européen des brevets

(11) **EP 3 928 753 B1**

(12) **EUROPEAN PATENT SPECIFICATION**

(45) Date of publication and mention
of the grant of the patent:
**18.09.2024 Bulletin 2024/38**

(21) Application number: **21173379.5**

(22) Date of filing: **11.05.2021**

(51) International Patent Classification (IPC):
*A61F 13/05* (2024.01)      *A61B 90/40* (2016.01)
*A61F 13/00* (2024.01)      *A61M 13/00* (2006.01)
*A61M 35/00* (2006.01)      *B01D 46/00* (2022.01)

(52) Cooperative Patent Classification (CPC):
**A61B 90/40; A61F 13/05; A61M 13/00;
A61M 35/30; B01D 46/00;** A61B 2090/401;
A61F 2013/0017; A61M 2202/0225;
A61M 2205/7518

(54) **DEVICES AND METHODS OF DELIVERING A GAS TO A WOUND SITE AND AN OPEN SURGICAL SITE**

VORRICHTUNGEN UND VERFAHREN ZUR ZUFÜHRUNG EINES GASES AN EINE WUNDSTELLE UND EINE OFFENE OPERATIONSSTELLE

DISPOSITIFS ET PROCÉDÉS D'ADMINISTRATION D'UN GAZ SUR UN SITE DE PLAIES ET UN SITE CHIRURGICAL OUVERT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.05.2020 US 202063029749 P**

(43) Date of publication of application:
**29.12.2021 Bulletin 2021/52**

(73) Proprietor: **Blacklock, Christopher Stephen
Sidlesham PO20 7LL (GB)**

(72) Inventor: **Blacklock, Christopher Stephen
Sidlesham PO20 7LL (GB)**

(74) Representative: **Buzzi, Notaro & Antonielli d'Oulx
S.p.A.
Corso Vittorio Emmanuele II, 6
10123 Torino (IT)**

(56) References cited:
**KR-A- 20090 038 854      RU-C2- 2 182 512
US-A- 5 487 889      US-A1- 2010 150 991
US-A1- 2018 318 566**

**Description**

Field of the Invention

The present invention relates to gas diffusion devices, also known as gas insufflators. In particular, this invention relates to a gas diffuser device used to cover a wound and deliver a gas to the wound site to enhance healing and reduce the potential for bacterial infections. The invention also relates to a gas diffuser device used to surround an open surgical site and deliver a gas to the surgical site. The invention also relates to systems for delivering the gas to the gas diffuser device and diffusing the gas through the device into the treatment site.

Background of the Invention

Wound care is desirable in order to improve health, enhance healing, and to reduce potential for infections of the outer epidermis, as well as underlying dermal and other tissues/organs. Wounds, either injury induced, or surgically induced, such as saphenous vein harvesting, require localized treatment to remedy the affected area and prevent further damage. If wounds are not properly treated, further complications can result, including wound irritation, secondary infections and further discomfort to the subject.

Improper wound care and/or wound healing result in greater costs and expenses, and may require antibiotic use, hospitalizations, and great pain or discomfort to the subject. Present methods of therapeutic wound care may still lead to higher than necessary infection rates and/or longer wound repair/recovery time. Thus, novel therapeutic methods and devices for wound care and wound healing are needed.

Therapeutic gases may be applied to the body for treatment of a variety of medical conditions. Carbon dioxide is a desirable therapeutic gas that has a bacteriostatic function, reducing the growth of bacteria and/or other microorganisms, which possibly may be present on or around medical treatment instruments, and at or around the wound or surgical site. Another desirable therapeutic effect of carbon dioxide gas is its high solubility rate in the tissues of the body relative to oxygen and nitrogen.

During operations which are performed in an open manner, i.e. when an inner portion of the body is uncovered for the performance of the surgical operation, it may be important to prevent air from the environment from reaching the open portion of the body. A gas diffuser (or gas insufflator) can be used to modify the local atmosphere around the operation by delivering the desired gas to the surgical site. Carbon dioxide is heavier than air so that a protective gas atmosphere in a volume adjoining an outwardly open, inner portion of a human being may be created in an easy manner. It is to be noted that the gas may be supplied to the volume in a continuous flow, wherein it is possible to ensure that the surrounding air is prevented from reaching the volume even if a part of the supplied gas leaves the area. During some surgical procedures, gas diffusers/insufflators deliver carbon dioxide gas into the open cavity of the surgical site to modify the local atmosphere in the open cavity so that it is as near to 100 percent $CO_2$ as possible. This modification of the local atmosphere has been shown to not only reduce the number of air emboli and therefore reduce the potential for a patient to suffer a stroke or organ damage from emboli, but to also have the potential to reduce infections.

US 2018/318566 discloses a gas diffuser device.

Gas diffuser/insufflators are known, but improved gas diffusers that provide a more convenient, inexpensive and accurate delivery of a therapeutic gas to the wound or surgical site while having the ability to maintain a stable local atmosphere of the therapeutic gas are needed. Further needed are gas diffuser/insufflators with improved ability to filter and deliver the therapeutic gas to the treatment site in order to enhance healing, reduce potential for bacterial infection and lessen the need for antibiotics.

Summary of the Invention

A gas diffuser device arranged to deliver a gas to a treatment site, the device being connectable to a gas source, the device including a gas inlet connectable to the gas source, (i) a first membrane comprising a flexible, hydrophobic, microporous polymer body having a pore size of 0.2 $\mu$m or less and (ii) a second membrane comprising a flexible nonporous or substantially nonporous polymer body. When the flexible, hydrophobic, microporous polymer body of the first membrane has a pore size of 0.2 $\mu$m or greater, the gas inlet has a bacterial filter.

Optionally, the second membrane may comprise a polymer having a pore size smaller than the pore size of the first membrane and/or a bacterial efficiency that is greater than the bacterial efficiency of the first membrane. The first and second membranes of the device each have outer edge portions and the outer edge portions of the second membrane are bonded to the outer edge portions of the first membrane to form an interior chamber of the device, the interior chamber of the device confining introduced gas to the interior chamber such that the introduced gas flows out through the pores of the first membrane. The gas is introduced into the interior chamber through a gas inlet of the device and the gas inlet is connectable to the gas source. The flexible, hydrophobic, microporous polymer body of the first membrane of the device is arranged to diffuse the introduced gas into the treatment site and the device is arranged such that the introduced gas maintains a gas atmosphere along the treatment site. The invention also provides a system including a gas source and such a device.

The invention also provides a gas diffuser device that includes a flexible, microporous polymer body having a pore size 0.2 µm or greater. Further, the pore size of the microporous polymer body could be in a range of 0.2 µm to 0.4 µm. Where the microporous polymer body of the first membrane has a pore size of 0.2 µm or greater, a bacterial filter is provided in the gas supply line or the gas inlet of the device to filter the gas supply. The invention also provides a system including a gas source and such a device.

It is to be understood that both the foregoing general description and the following detailed description are exemplary and explanatory and are intended to provide further explanation of the invention as claimed.

Brief Description of the Drawings

Preferred forms of the present invention will now be described by way of examples with reference to the accompanying drawings.

    FIG. 1 shows a device of the invention in cross-section attached to a gas supply line and a gas source.
    FIG. 2 shows a view of the wound covering surface of the device.
    FIG. 3 shows a view of the non-wound covering surface of the device.
    FIG. 4 shows a view of the device of the invention (in cross-section) and system in use for treating a wound.
    FIG. 5 shows a view of the device of the invention (in cross-section) and system in use for treating an open surgical site.
    FIG. 6 shows and alternate embodiment of the device of the invention.

Detailed Description of the Preferred Embodiments

In one embodiment the invention is a device arranged to deliver a gas to a treatment site, the device being connectable to a gas source, the device including (i) a first membrane comprising a flexible, hydrophobic, microporous polymer body (ii) a second membrane comprising a flexible nonporous or substantially nonporous polymer body. Optionally, the second membrane may comprise a polymer having a pore size smaller than the pore size of the first membrane. The first and second membranes of the device each have outer edge portions and the outer edge portions of the second membrane are bonded to the outer edge portions of the first membrane to form an interior chamber of the device, the interior chamber of the device confining introduced gas to the interior chamber such that the introduced gas flows out through the pores of the first membrane. The gas is introduced into the interior chamber through a gas inlet of the device and the gas inlet is connectable to the gas source. The flexible, hydrophobic, microporous polymer body of the first membrane of the device is arranged to diffuse the introduced gas into the treatment site and the device is arranged such that the introduced gas maintains a gas atmosphere along the treatment site.

In another embodiment the invention is a device arranged to deliver a gas to a treatment site, the device being connectable to a gas source, the device including (i) a first membrane comprising a flexible, hydrophobic, microporous polymer body having a pore size of 0.2 µm or less and (ii) a second membrane comprising a flexible nonporous or substantially nonporous polymer body. Optionally, the second membrane may comprise a polymer having a pore size smaller than the pore size of the first membrane. The first and second membranes of the device each have outer edge portions and the outer edge portions of the second membrane are bonded to the outer edge portions of the first membrane to form an interior chamber of the device, the interior chamber of the device confining introduced gas to the interior chamber such that the introduced gas flows out through the pores of the first membrane. The composition of the material of the second membrane prevents or inhibits the gas from escaping through the second membrane and directs or forces the gas through the pores of the first membrane. The gas is introduced into the interior chamber through a gas inlet of the device and the gas inlet is connectable to the gas source. The flexible, hydrophobic, microporous polymer body of the first membrane of the device is arranged to diffuse the introduced gas into the treatment site and the device is arranged such that the introduced gas maintains a gas atmosphere along the treatment site.

The advantage of letting the gas pass through the microporous polymer body of the first membrane having a pore size of 0.2 µm or less is that the pores of the microporous polymer body, which are great in number and positioned very closely to each other, filter bacteria that may present. The average diameter of spherical bacteria is 0.5-2.0 µm. For rod-shaped or filamentous bacteria, the average length is 1-10 µm and the average diameter is 0.25-1.0 µm. Specific examples of bacteria include: *E. coli* having an average size of about 1.1 to 1.5 µm wide by 2.0 to 6.0 µm long; Spirochetes ranging from 3 to 500 µm in length; the cyanobacterium *Oscillatoria* being about 7 µm in diameter. Additionally, one group of bacteria called the mycoplasmas, have individuals with size much smaller than these dimensions. They can measure about 0.25 µm and are the smallest cells known so far. They were formerly known as pleuropneumonia-like organisms (PPLO). *Mycoplasma gallicepticum,* with a size of approximately 200 to 300 nm are thought to be the world smallest bacteria. Therefore a microporous polymer body that has a microporous polymer body having pore sizes of 0.2 µm or less will thus filter and remove most/almost all bacteria from the gas supply. The microporous polymer body of the first membrane a pore size of 0.2 µm or less can also filter other microorganisms, impurities, or other foreign substances that also may be present.

Another advantage of letting the gas passing through the microporous polymer body of the first membrane is that the pores can function as a multiplicity of supply nozzles, and may distribute the gas in thin layers lying close to each other and forming, when the gas leaves the microporous polymer body, a substantially laminar continuous gas flow. The flexible, hydrophobic, microporous polymer body of the first membrane also causes the gas to exit through pores over the majority of the body thereby preventing a singular jetting action.

Diffusion of the gas, preferably carbon dioxide, through the microporous first membrane into the treatment site is advantageous because carbon dioxide has a high solubility in the tissue of the body relative to oxygen and nitrogen, has a bacteriostatic function, which reduces the growth of bacteria and/or other microorganisms and is heavier than air. These features of carbon dioxide aid in the enhancement of healing at the treatment site; aid in the reduction of the potential for bacterial infections at the treatment site; lessen the need for antibiotics at the treatment site; and aids in the creation of a therapeutic and protective gas atmosphere at the treatment site.

In another embodiment the first membrane of the device may include a flexible, microporous polymer body having a pore size of 0.2 $\mu$m or greater, and that could be in a range of 0.2 $\mu$m to 0.4 $\mu$m. In applications where the microporous polymer body of the first membrane has a pore size larger than 0.2 $\mu$m, a bacterial filter is provided in the gas supply line or the gas inlet of the device to filter the gas supply.

In an embodiment the introduced gas is $CO_2$. In another embodiment the device is arranged to attach to the treatment site by an adhesive positioned on the first membrane, by an adhesive positioned on the second membrane, or both. In an embodiment the device has edges and the device has a shape formed from the edges of the device, the shape being rectangular, square, triangular, oval, trapezoidal or circular The flexible, microporous polymer body of the first membrane is hydrophobic. In an embodiment the flexible, microporous polymer body of the first membrane is made of polytetrafluoroethylene and the flexible, nonporous or substantially nonporous polymer body of the second membrane is also made of a polytetrafluoroethylene. In another embodiment the flexible, microporous polymer body of the first membrane is coated with an antibacterial substance and a medicated substance.

In another embodiment the device includes a pressure relief valve whereby introduced gas in the interior chamber of the device can be diverted from the interior chamber and through the pressure relief valve to maintain a desired gas pressure along the treatment site. In an embodiment the device includes a flush line port whereby introduced gas in the interior chamber of the device can be diverted from the interior chamber and through the flush line port to flush the introduced gas through the device. In another embodiment the device includes an open cell sponge support material, the open cell sponge support material being positioned in the interior chamber in direct contact with the flexible, microporous polymer body of the first membrane. In another embodiment the device includes shapeable structures positioned throughout the device and arranged to conform and shape the device around the treatment site.

The invention also provides a system comprising a gas source and such a device having a first membrane with a microporous polymer body having a pore size of 0.2 $\mu$m or less. The gas insert of the device can be connected directly to the gas source or can be connected to a flexible hose portion (or other gas delivery means) that is then connected to the gas supply. In some embodiments of the system, the microporous polymer body of the first membrane of the device may have a a pore size larger than 0.2 $\mu$m. In such embodiments of the system, a filter is provided in the flexible hose portion (or other gas delivery means) or the gas inlet of the device to filter the gas supply. The filter positioned in the gas inlet or the flexible hose portion may have a housing made of polypropylene and may have glass fibers as a filter material. The filter may have a pore size between 0.1 to 0.4 $\mu$m. It is to be understood that the filter could be made of any desired material and could have any desired pore size as needed to properly and adequately filter the gas supply. In an embodiment, the gas comprises a majority of carbon dioxide.

Also disclosed but not part of the invention is a method for delivering a gas to a treatment site including providing a device arranged to deliver gas to a treatment site, the device being connectable to a gas source, the device having (i) a first membrane comprising a flexible, microporous polymer body having a bacterial filtration efficiency of 99.9% or greater and/or a pore size of 0.2 $\mu$m or less and (ii) a second membrane comprising a flexible nonporous or substantially nonporous polymer body. Optionally, the second membrane may comprise a polymer having a pore size smaller than the pore size of the first membrane and/or a bacterial efficiency that is greater than the bacterial efficiency of the first membrane. The first and second membranes of the device each have outer edge portions and the outer edge portions of the second membrane are bonded to the outer edge portions of the first membrane to form an interior chamber of the device. The composition of the material of the second membrane prevents or inhibits the gas from escaping through the second membrane and directs or forces the gas through the pores of the first membrane. The method not according to the invention including positioning and attaching the device to the treatment site; connecting the gas inlet of the device to the gas source; and supplying the gas to the interior chamber of the device through the gas inlet, the interior chamber of the device confining the supplied gas such that the supplied gas flows out through the pores of the first membrane of the device and diffuses the supplied gas into the treatment site. The method not according to the invention includes that the supplied gas maintains a gas atmosphere along the treatment site.

In some embodiments not according to the invention of the method, the microporous polymer body of the first membrane of the device may have a bacterial filtration efficiency of less than 99.9% and/or a pore size larger than 0.2 μm. In such embodiments, not according to the invention the first membrane could have a bacterial filtration efficiency of 99.0% or greater, 99.5% or greater or could be in a range of 99.0% to 99.9%. Additionally/alternatively the first membrane could have a pore size in a range of 0.2 μm to 0.4 μm. In such embodiments of the method, not according to the invention a bacterial filter may be provided in the flexible hose portion of the gas source or the gas inlet of the device to filter the gas supply. In an embodiment, the gas comprises a majority of carbon dioxide.

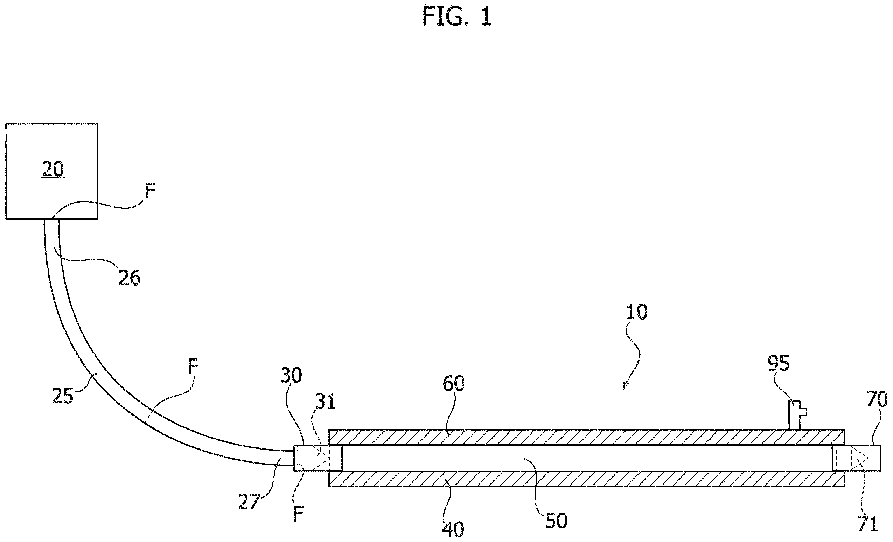

FIG. 1 shows a gas diffuser or insufflator device 10 of the invention (in cross-section connected to a gas source 20. The gas diffuser device 10 has gas inlet 30, first membrane 40, interior chamber 50, second membrane 60 and flush line outlet or port 70 (optional). Gas diffuser device 10 may also have an overpressure relief valve 95. Gas may be supplied from the gas source to the device by flexible gas hose 25 that has intake end 26 connected to gas source 20 and discharge end 27 connected to the gas inlet 30 of the gas diffuser device 10. It should be understood that any desired gas delivery structure or means can be used to supply gas to the device.

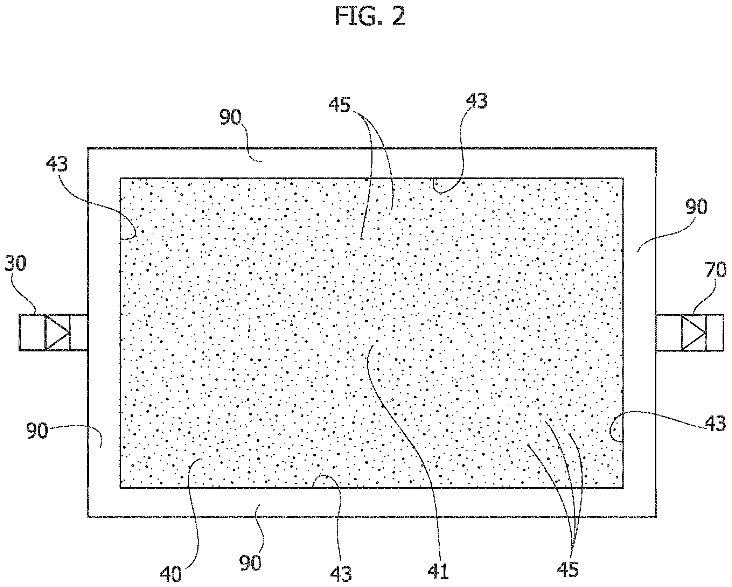

FIG. 2 shows a view of the wound covering surface of gas diffuser 10. First membrane 40 is a microporous membrane through which a supplied gas is filtered and diffused to a treatment site. The treatment site may be a wound or surgical site depending upon the application. First membrane 40 may be made of a flexible microporous polymer such as polytetrafluoroethylene and, more specifically, could be made of POREX® Virtek™ PTFE MD10. It is to be understood that the material composition of first membrane 40 is non-limiting and could be any microporous polymer that filters bacteria and other impurities as discussed further below. First membrane 40 may have a thickness as desired and could be 0.13 mm. First membrane 40 is hydrophobic and non-wetting. First membrane 40 has pores 45 positioned close together across the first membrane 40 and are distributed across the first membrane in great number and high concentration. Pores 45 prevent the passage of bacteria and other microbes in addition to other impurities larger than the size of the pore from diffusing through first membrane 40 to the treatment site, thereby filtering the gas supplied or introduced to the device. Thus, first membrane 40 can function as a bacterial filter allowing the desired gas to pass through pores 45 while preventing bacteria from passing through pores 45. The bacterial filtration property of first membrane 40 can have a desired efficiency of bacterial filtration. The bacterial filtration efficiency of the flexible, microporous polymer body of the first membrane 40 can be determined/measured in accordance with ASTM F2101-19. The desired bacterial efficiency of first membrane 40 as determined by ASTM F2102-19 could be 99.9% or greater, 99.0% or greater, 99.5% or greater or could be in a range of 99.0% to 99.9% as desired. (It should be understood that the bacterial filtration efficiency could also be less than 99.0% depending upon the application). In some applications where the bacterial filtration efficiency of first membrane 40 is less than 99.9%, a separate filter (F shown with optional locations in FIG.1) is provided to the gas source, gas supply line or the gas inlet of the gas diffuser device to filter out bacteria and other impurities.

Optionally, pores 45 of first membrane 40 may have a specific desired size. The desired size could be 0.2 microns or less, the desired pore size could be 0.2 microns or greater, or the desired pore size could be in a range of 0.2 μm to 0.4 μm. Pores 45 that are sized 0.2 microns or less can function as a bacterial filter since the average diameter of spherical bacteria is 0.5-2.0 μm, and for rod-shaped or filamentous bacteria, the average length is 1-10 μm and the average diameter is 0.25-1.0 μm. In some applications, the first membrane 40 will have pores 45 sized larger than 0.2 microns and, in those circumstances, a separate filter (F shown with optional locations in FIG. 1) may be provided to the gas source, gas supply line or the gas inlet of the gas diffuser device to filter out bacteria and other impurities.

First membrane 40 may have body portion 41 surrounded by edge portions 43. Body portion 41 and edge portions 43 may all have pores 45. The gas diffusion device 10 can include adhesive surfaces 90, which may be covered by release paper. Adhesive surfaces 90 adhere to the patient's body surrounding the treatment site. Adhesive surfaces 90 could be an adhesive coating applied to the device or could be a separate film that is attached to or covers the device.

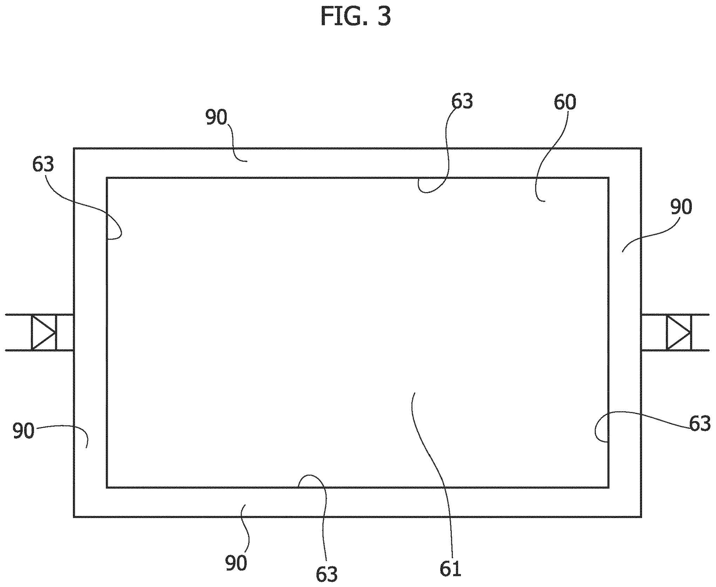

As seen in FIGS. 1 and 3, second membrane 60 may be made of any desired material and could be made from a polymer, a thin metal foil, surgical steel, leather and/ or any material that will hold its structural integrity during use and is capable of being sterilized. Second membrane 60 may be flexible or in some embodiments may not be flexible as to hold its shape during usage. The second membrane could also be made out of the same polymer as the first membrane 40, such as a polytetrafluoroethylene and, more specifically, could be made of POREX® Virtek™ PTFE MD10. The second membrane may have any thickness as desired and could be 0.13 mm, and may also be hydrophobic and non-wetting. Second membrane 60 may be nonporous or substantially nonporous. The second membrane 60 may have a pore size smaller than the pore sizes (in some applications much smaller) of first membrane 40 and/or a bacterial efficiency rate that is greater than the bacterial efficiency rate of first membrane 40, thereby directing the flow of gas through gas diffuser 10 and through first membrane 40 while preventing or decreasing (in some applications greatly decreasing) the escape or diffusion of gas through the second membrane. As such, the smaller pore size and/or the greater bacterial efficiency rate of second membrane 60 allows for the second membrane to be nonporous or substantially nonporous, as compared to first membrane 40. In an optional embodiment, second membrane 60 may be made of a number of sheets or layers of a porous material that are bonded/adhered/affixed to one another to make the second membrane 60 less porous or substantially nonporous. In an alternative embodiment, second membrane may be made from the same material as first membrane 40 with a backing sheet affixed to it for stopping the flow of gas through second membrane 60 until such a time that the wound is required to breath or be observed. At this time the backing sheet can be removed and the wound can breath, be allowed to dry and be observed.

Second membrane 60 may have body portion 61 surrounded by edge portions 63. Edge portions 63 of second membrane 60 are bonded/secured/adhered to edge portions 43 of first membrane by sonic welding, thermal welding, compression sealing, induction heating, adhesive or other processes known in the art. The bonding of outer edge portions 63 of the second membrane 60 to the outer edge portions 43 of the first membrane 40 form interior chamber 50 of the device. Second membrane 60 can prevent or inhibit gas introduced into the interior chamber from escaping. The second membrane 60 confines/directs the flow of the introduced gas in interior chamber 50 such that the introduced gas flows out or diffuses through the pores 45 of the first membrane 40 to the treatment site. Some sections of outer edge portions 63 of second membrane 60 and outer edge portions 43 of first membrane 40 are bonded/secured/adhered to portions of gas inlet 30 and optional flush line outlet or port 70. The device 10 may include adhesive surfaces 90, which may be covered by release paper and adhere to the patient's body surrounding the treatment site. Adhesive surfaces 90 can be an adhesive coating applied to the device or to either/both of the first and second membranes. Alternatively, adhesive surfaces 90 can also be a separate film that is attached to the device or to either/both of the first and second membranes.

Gas inlet 30 of device 10 can connect to the flexible gas hose (or other gas delivery means) and to the gas source through various means as known in the art. For example, a barbed or smooth push fitting could be used to connect the gas inlet to the gas supply hose for delivery of $CO_2$ when it is deemed that the connection to the supply of gas should be connected for longer term care. Additionally, there is also the option of having quick disconnect fittings and screw fittings for supplying smaller durations of $CO_2$ to the device that allow for easier/quicker disconnection of the gas delivery means. In order to maintain the therapeutic gas atmosphere along the treatment site and inside the device, the gas inlet may have a one way valve 31 to prevent gas that enters the device through gas inlet 30 from escaping back through the gas inlet. Alternatively/additionally, a Halkey-Roberts clamp, a plug, a cap or a tap could be used to prevent gas from escaping the device back through the gas inlet.

Optional flush line outlet or port 70 may be provided so that the gas delivered to the gas diffusion device can be flushed through the device, thereby removing gases/air, condensation, fluids or impurities that may exist at the treatment site and that may have built up within the device during use. The flush line outlet 70 may have a one way valve 71 to allow gas within the device to have a controlled exit or release from the device through the flush line outlet. The one way valve prevents any uncontrolled gas/air that is outside the device from entering into the device. Alternatively/additionally, a Halkey-Roberts clamp, a plug, a cap or a tap could be used to prevent gas from entering the device through the flush line outlet. The flush line outlet may have an option of having a three way tap on it. The open end of the purge line may have a female luer connection to allow a syringe to be attached when required to draw off any gas or fluid as required from the device and treatment site.

Gas diffuser device 10 may also have an overpressure relief valve 95 (optional) attached thereto allowing gas to be released or purged from the device when the pressure inside the device becomes too high and passes a desired threshold, thereby reducing the risk of damaging the device and potentially the treatment site.

Pore size of the flexible microporous polymer membrane can be determined using the Mercury Intrusion Method. In a vacuum, a mercury drop will not enter a pore due to its very high surface tension, but will if pressure is applied. It is known that, for a given pore size, a certain pressure is required to force the mercury into the pore. For each incremental increase in pressure, the change in intrusion volume is equal to the volume of the pores whose diameters fall within an interval that corresponds to the particular pressure interval. The amount of displaced mercury can therefore be used to calculate the pore size using a graphical representation. The pore size will be the average size of the pore distribution obtained (i.e. the peak value).

The Washburn Equation can be used to convert pressure to pore diameter:

$$D = -4y \, (\cos\theta)/P$$

where D = Diameter of pore being intruded
y = Surface tension of mercury
P = Intrusion pressure
θ = contact angle between mercury & material

For example, to arrive at a pore size in $\mu$m, y is 480 N/m, θ is in degrees, and P is the intrusion pressure, the pressure at which 50% of the volume of mercury intrudes into the pores. The cumulative volume starts at zero and pressure is applied until no more mercury can be introduced (giving total volume of the pores at this point). In a typical test, a graph of cumulative volume ($mm^3$/g) versus intrusion pressure (kPa) is made. The intrusion pressure is then read off the graph. This is the "50% value". It means that 50% of the pores lie above this diameter and 50% lie below it. Pore size in this application, including the claims, means this 50% value, with 50% of the pores being above this diameter and 50% being below it.

The pores 45 of the microporous flexible polymer first membrane 40 allow the gas, preferably $CO_2$, to diffuse over the full surface area of the membrane. The small pore size means that even at flows as low at 2.5 liters per minute (LPM) it will still act as a very efficient gas diffuser. The smaller pore size means in effect that the gas has to make more effort to exit the microporous flexible polymer first membrane 40 thereby flowing through more pores 45. As such, an almost instantaneous therapeutic gas atmosphere can be formed along the treatment site once the gas is introduced through positive pressure to the gas diffusion device 10. The introduced gas within the interior chamber of the gas diffusion device can have a higher velocity than the gas along the treatment site that has been diffused through the pores 45 of first membrane 40. Thus the flow/diffusion of the introduced gas through the pores of the first membrane can create a slow, substantially laminar, gas flow whereby turbulence of the gas atmosphere is minimized.

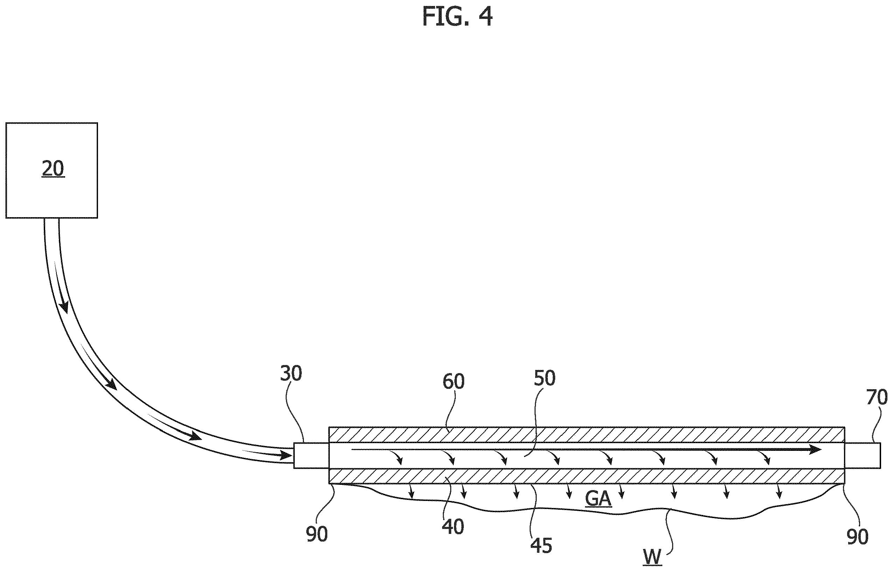

FIG. 4 shows gas diffusion device 10 (in cross-section) in use for treating a wound W. Adhesion surfaces 90 are secured/affixed to the patient's body such that the device covers and surrounds the wound to be treated. Gas flows via positive pressure from gas source 20 through flexible hose 25, through gas inlet 30, and into interior chamber 50 of the gas diffusion device 10 (gas flow and diffusion across first membrane 40 shown with directional arrows). The gas, which may be carbon dioxide, flows out through the multiplicity of pores 45 of flexible, microporous polymer first membrane 40 (pores 45 are indicated in FIG. 2 but are too small to actually be seen) into the treatment site of the wound creating a gas atmosphere GA along the treatment site and wound. The gas may flow at any desired rate as to allow proper and adequate diffusion along the treatment site and could, for example, have a flow rate of greater than 0.0001 l/hr/cm² Δp 70mbar. In some embodiments, the gas (and other impurities) can be flushed out from the device through optional flush line outlet 70. In some embodiments gas delivered to the device having a pressure higher than a desired threshold can be purged or released through optional overpressure valve 95, thereby protecting the device and the wound from damage.

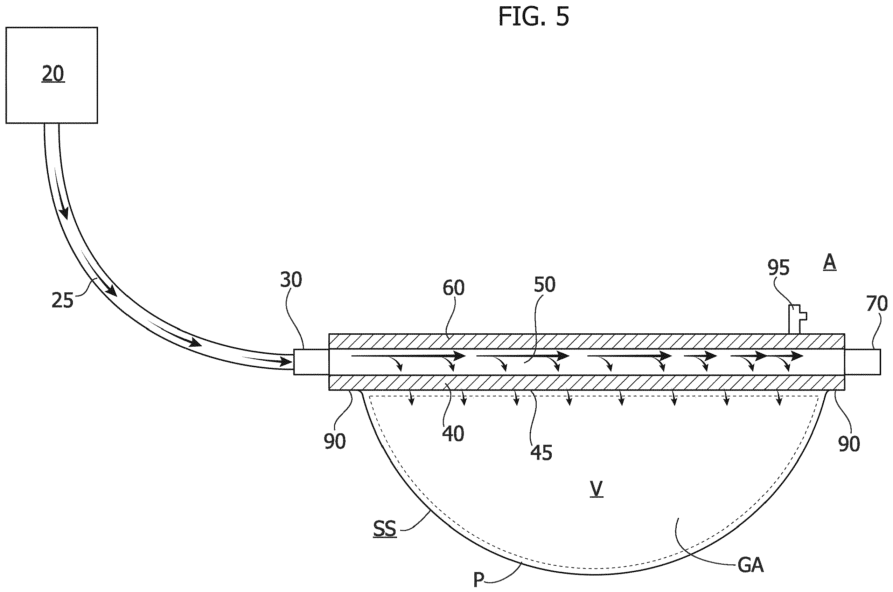

FIG. 5 shows gas diffusion device 10 in use for treating an open surgical site SS. Adhesion surfaces 90 are secured to the patient's body around open volume V and adjacent to a portion P of a human body that is normally not exposed to the atmosphere, as in a surgery. Gas flows via positive pressure from gas source 20 through flexible hose 25, through gas inlet 30, into interior chamber 50 of the gas diffusion device 10 (gas flow and diffusion across first membrane 40 shown with directional arrows). The gas, preferably carbon dioxide, flows out through the multiplicity of pores 45 of flexible, microporous polymer first membrane 40 (pores 45 are indicated in FIG. 2 but are too small to actually be seen) which fills the volume V forming a protective therapeutic gas atmosphere GA and preventing air A from the environment from reaching the volume. As CO2, the preferred gas, is heavier than air, the CO2 will accumulate in the volume V as long as the gas flow into the volume V is not turbulent. In some embodiments, the gas (and other impurities) can be flushed out from the device through optional flush line outlet 70. In some embodiments gas delivered to the device having a pressure higher than a desired threshold can be purged or released through optional overpressure valve 95, thereby protecting the device and the wound from damage.

In order to prevent air embolism, i.e., a blocking of the capillaries and small vessels which may be caused by an air bubble, the therapeutic gas atmosphere in a volume adjoining a temporarily, outwardly open portion of a human being ought to include a delivered gas, the majority of the gas being carbon dioxide. In the applications where a therapeutic gas atmosphere is to be created in a volume adjoining an outwardly open inner portion of the body of a human being or an animal, it is advantageous that the gas includes carbon dioxide due to the fact that carbon dioxide has a high solubility in the tissue of the body relative to oxygen and nitrogen and because carbon dioxide is heavier than air. It is to be noted that the gas may be supplied to the volume in a continuous flow, wherein it is possible to ensure that the surrounding air is prevented from reaching the volume even if a part of the supplied gas leaves the area. Another possibility is, at least initially, to supply gas continuously in order to create therapeutic gas atmosphere, and then supply gas periodically to maintain the gas atmosphere. It should also be noted that the gas may include oxygen, for instance in the cases when said tissue of said open body portion is strongly oxygen dependent. Oxygen, as well as carbon dioxide, is heavier than air so that the protecting atmosphere in the volume may be created in an easy manner since the heavier gas will pass downwardly in the open body portion and force away the non-sterile air present in the lower part of this open portion. In certain applications a protecting atmosphere including sterile air may be satisfactory. The main thing is that air from the environment, i.e., non-sterile air, is prevented from reaching the volume.

In one embodiment, the gas diffusion device will include a flexible shapeable gas delivery tube or hose extending from the gas inlet through the interior chamber of the gas diffusion device. The flexible, shapeable gas delivery tube includes multiple perforations or pores. The shapeable tube will allow the gas diffusion device to be shaped so that in certain instances it can surround the surgical site.

In one embodiment, the gas diffusion device will include shapeable structures within the first and second membranes and interior chamber which will allow the gas diffusion device to be molded around a surgical site in order to create the gas atmosphere.

In one embodiment, the microporous first membrane can also be coated in an antibacterial substance and also a medicated substance to aid healing and reduce pain and infections.

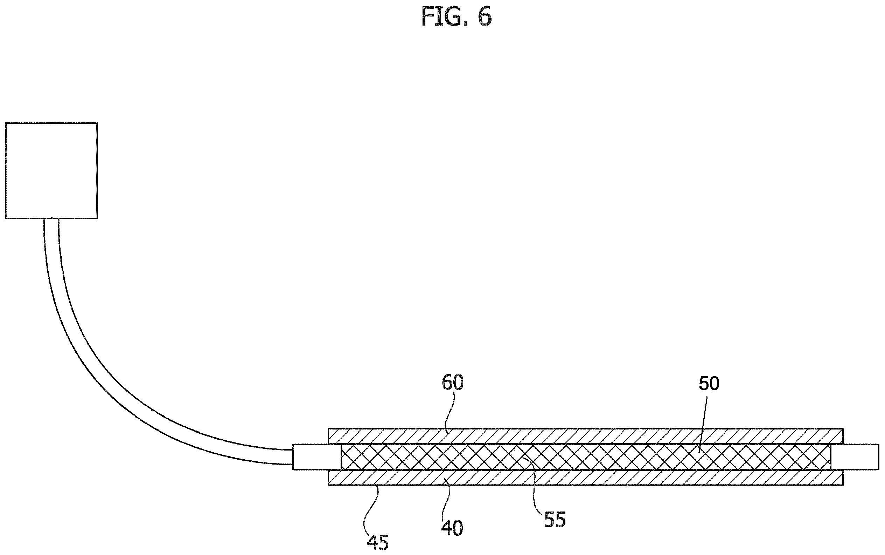

In one embodiment shown in FIG. 6, the gas diffusion device will include an open cell sponge support material 55 positioned along the first membrane inside the interior chamber 50 of the device. The open cell sponge support material will aid the $CO_2$ gas in diffusing along the majority of the first membrane 40 and through pores 45. In another embodiment the support material inside the interior chamber could have baking soda or another similar chemical compound impregnated into the support material 55 between the first and second membrane. An acidic fluid such as vinegar, rather than a gas could be supplied through the inlet of the device. The acidic fluid in contact with the baking soda or other similar compound causes a chemical reaction whereby carbon dioxide is created. The carbon dioxide is then diffused to the treatment site through pores 45 located in the microporous first membrane 40.

Although particular embodiments have been disclosed herein in detail, this has been done for purposes of illustration only, and is not intended to be limiting with respect to the scope of the following appended claims. In particular, it is contemplated by the inventors that various substitutions, alterations, and modifications may be made to the invention without departing from the scope of the invention as defined by the claims.

**Claims**

1. A device (10) arranged to deliver a gas to a treatment site, the device being connectable to a gas source (20), the device (10) comprising:

   - a gas inlet (30) connectable to the gas source (20),
   - a first membrane (40) comprising a flexible, hydrophobic, microporous polymer body (41), wherein the flexible, hydrophobic, microporous polymer body (41) has one of the following features:

      (a) a pore size of 0.2 μm or less, or
      (b) a pore size of 0.2 μm or greater, and

   - a second membrane (60) comprising a flexible polymer body (61),
   wherein when the flexible, hydrophobic, microporous polymer body of the first membrane (40) has feature (b) the gas inlet (30) has a bacterial filter (F),
   wherein the first (40) and second (60) membranes each have outer edge portions (43, 63) and the outer edge portions (63) of the second membrane are bonded to the outer edge portions (43) of the first membrane (40) to form an interior chamber (50) of the device (10), the interior chamber (50) of the device confining introduced gas to the interior chamber (50) such that the introduced gas flows out through pores (45) of the first membrane (40), the gas being introduced into the chamber (50) through the gas inlet (30) of the device (10),
   wherein the flexible, hydrophobic, microporous polymer body of the first membrane (40) is arranged to diffuse the introduced gas into the treatment site and
   wherein the introduced gas maintains a gas atmosphere along the treatment site.

2. The device (10) according to claim 1, wherein the introduced gas is $CO_2$.

3. The device (10) according to any one of the preceding claims, wherein the device (10) is arranged to attach to the treatment site.

4. The device (10) according to any one of the preceding claims, wherein the device (10) is attached to the treatment site by an adhesive (90) positioned on the first membrane (40) or on the second membrane (60).

5. The device (10) according to any one of the preceding claims, wherein the device (10) has edges and wherein the device (10) has a shape formed from the edges of the device (10), the shape being rectangular, square, triangular, oval, trapezoidal or circular.

6. The device (10) according to any one of the preceding claims, wherein the flexible, microporous polymer body (41) of the first membrane (40) has at least one of the following features:

   - it is made of polytetrafluoroethylene, and
   - it is coated with an antibacterial substance and a medicated substance.

7. The device (10) according to any one of the preceding claims, wherein the flexible polymer body (61) of the second membrane (60) has at least one of the following features:

   - it is nonporous, and
   - it is made of polytetrafluoroethylene.

8. The device (10) according to any one of the preceding claims, wherein the device (10) further comprises at least one of:

   - a pressure relief valve (95), so that the introduced gas in the interior chamber (50) of the device (10) can be diverted from the interior chamber (50) and through the pressure relief valve (95) to maintain a desired gas pressure along the treatment site;

- a flush line port (70), so that the introduced gas in the interior chamber (50) of the device (10) can be diverted from the interior chamber (50) and through the flush line port (70) to flush the introduced gas through the device (10);

- an open cell sponge support material (55) positioned in the interior chamber (50) in direct contact with the flexible, microporous polymer body of the first membrane (40); and

- shapeable structures positioned throughout the device (10) and arranged to conform the device (10) around the treatment site.

9. The device (10) according to any one of the preceding claims, wherein the treatment site is a wound or a surgical site.

10. The device (10) according to any one of the preceding claims, wherein, when the flexible, hydrophobic, microporous polymer body of the first membrane (40) has a pore size of 0.2 µm or greater, the flexible, hydrophobic, microporous polymer body of the first membrane (40) has a pore size in the range of 0.2 µm to 0.4 µm.

11. A system comprising a gas source (20) and a device (10) according to any one of claims 1 to 10.

**Patentansprüche**

1. Vorrichtung (10), die so eingerichtet ist, dass sie ein Gas an eine Behandlungsstelle liefert, wobei die Vorrichtung mit einer Gasquelle (20) verbindbar ist, wobei die Vorrichtung (10) umfasst:

- einen Gaseinlass (30), der mit der Gasquelle (20) verbindbar ist,
- eine erste Membran (40), die einen flexiblen, hydrophoben, mikroporösen Polymerkörper (41) umfasst, wobei der flexible, hydrophobe, mikroporöse Polymerkörper (41) eines der folgenden Merkmale aufweist:

(a) eine Porengröße von 0,2 µm oder weniger oder
(b) eine Porengröße von 0,2 µm oder mehr, und

- eine zweite Membran (60), die einen flexiblen Polymerkörper (61) umfasst, wobei, wenn der flexible, hydrophobe, mikroporöse Polymerkörper der ersten Membran (40) das Merkmal (b) aufweist, der Gaseinlass (30) ein Bakterienfilter (F) aufweist, wobei die erste (40) und die zweite (60) Membran jeweils Außenrandabschnitte (43, 63) aufweisen und die Außenrandabschnitte (63) der zweiten Membran mit den Außenrandabschnitten (43) der ersten Membran (40) verbunden sind, um eine Innenkammer (50) der Vorrichtung (10) zu bilden, wobei die Innenkammer (50) der Vorrichtung in die Innenkammer (50) eingeleitetes Gas derart beschränkt, dass das eingeleitete Gas durch Poren (45) der ersten Membran (40) ausströmt, wobei das Gas in die Kammer (50) durch den Gaseinlass (30) der Vorrichtung (10) eingeleitet wird, wobei der flexible, hydrophobe, mikroporöse Polymerkörper der ersten Membran (40) so angeordnet ist, dass er das eingeleitete Gas in die Behandlungsstelle diffundiert, und wobei das eingeleitete Gas eine Gasatmosphäre entlang der Behandlungsstelle aufrechterhält.

2. Vorrichtung (10) nach Anspruch 1, wobei es sich bei dem eingeleiteten Gas um $CO_2$ handelt.

3. Vorrichtung (10) nach einem der vorhergehenden Ansprüche, wobei die Vorrichtung (10) so eingerichtet ist, dass sie an der Behandlungsstelle angebracht werden kann.

4. Vorrichtung (10) nach einem der vorhergehenden Ansprüche, wobei die Vorrichtung (10) durch ein Haftmittel (90), das sich auf der ersten Membran (40) oder auf der zweiten Membran (60) befindet, an der Behandlungsstelle angebracht wird.

5. Vorrichtung (10) nach einem der vorhergehenden Ansprüche, wobei die Vorrichtung (10) Ränder aufweist und wobei die Vorrichtung (10) eine aus den Rändern der Vorrichtung (10) gebildete Form aufweist, wobei die Form rechteckig, quadratisch, dreieckig, oval, trapezförmig oder kreisförmig ist.

6. Vorrichtung (10) nach einem der vorhergehenden Ansprüche, wobei der flexible, mikroporöse Polymerkörper (41) der ersten Membran (40) mindestens eines der folgenden Merkmale aufweist:

- er ist aus Polytetrafluorethylen hergestellt, und
- er ist mit einer antibakteriellen Substanz und einer medikamentösen Substanz beschichtet.

7. Vorrichtung (10) nach einem der vorhergehenden Ansprüche, wobei der flexible Polymerkörper (61) der zweiten Membran (60) mindestens eines der folgenden Merkmale aufweist:

- er ist nichtporös, und
- er ist aus Polytetrafluorethylen hergestellt.

**8.** Vorrichtung (10) nach einem der vorhergehenden Ansprüche, wobei die Vorrichtung (10) ferner mindestens eines der Folgenden umfasst:

- ein Druckentlastungsventil (95), so dass das in die Innenkammer (50) der Vorrichtung (10) eingeleitete Gas aus der Innenkammer (50) und durch das Druckentlastungsventil (95) abgeleitet werden kann, um einen gewünschten Gasdruck entlang der Behandlungsstelle aufrechtzuerhalten;
- eine Spülleitungsöffnung (70), so dass das in die Innenkammer (50) der Vorrichtung (10) eingeleitete Gas aus der Innenkammer (50) und durch die Spülleitungsöffnung (70) abgeleitet werden kann, um das eingeleitete Gas durch die Vorrichtung (10) zu spülen;
- ein offenzelliges Schwammstützmaterial (55), das in der Innenkammer (50) in direktem Kontakt mit dem flexiblen, mikroporösen Polymerkörper der ersten Membran (40) angeordnet ist; und
- formbare Strukturen, die über die gesamte Vorrichtung (10) verteilt und so eingerichtet sind, dass sie die Vorrichtung (10) um die Behandlungsstelle herum anpassen.

**9.** Vorrichtung (10) nach einem der vorhergehenden Ansprüche, wobei die Behandlungsstelle eine Wunde oder eine Operationsstelle ist.

**10.** Vorrichtung (10) nach einem der vorhergehenden Ansprüche, wobei, wenn der flexible, hydrophobe, mikroporöse Polymerkörper der ersten Membran (40) eine Porengröße von 0,2 $\mu$m oder mehr aufweist, der flexible, hydrophobe, mikroporöse Polymerkörper der ersten Membran (40) eine Porengröße im Bereich von 0,2 $\mu$m bis 0,4 $\mu$m aufweist.

**11.** System, das eine Gasquelle (20) und eine Vorrichtung (10) nach einem der Ansprüche 1 bis 10 umfasst.

**Revendications**

**1.** Dispositif (10) agencé pour administrer un gaz sur un site de traitement, le dispositif pouvant être relié à une source de gaz (20), le dispositif (10) comprenant :

une entrée de gaz (30) pouvant être reliée à la source de gaz (20),
une première membrane (40) comprenant un corps en polymère microporeux hydrophobe souple (41), dans lequel le corps en polymère microporeux hydrophobe souple (41) présente l'une des caractéristiques suivantes :

(a) une dimension des pores de 0,2 $\mu$m ou moins, ou
(b) une dimension des pores de 0,2 $\mu$ ou plus ; et

une seconde membrane (60) comprenant un corps en polymère souple (61),
dans lequel lorsque le corps en polymère microporeux hydrophobe souple de la première membrane (40) présente la caractéristique (b), l'entrée de gaz (30) a un filtre bactérien (F),
dans lequel les première (40) et seconde (60) membranes ont chacune des parties de bord externe (43, 63) et les parties de bord externe (63) de la seconde membrane sont liées aux parties de bord externe (43) de la première membrane (40) pour former une chambre intérieure (50) du dispositif (10), la chambre intérieure (50) du dispositif confinant un gaz introduit dans la chambre intérieure (50) de sorte que le gaz introduit s'écoule dehors à travers des pores (45) de la première membrane (40), le gaz étant introduit dans la chambre (50) par l'intermédiaire de l'entrée de gaz (30) du dispositif (10),
dans lequel le corps en polymère microporeux hydrophobe souple de la première membrane (40) est agencé pour diffuser le gaz introduit dans le site de traitement et dans lequel le gaz introduit maintient une atmosphère gazeuse dans tout le site de traitement.

**2.** Dispositif (10) selon la revendication 1, dans lequel le gaz introduit est le $CO_2$.

**3.** Dispositif (10) selon l'une quelconque des revendications précédentes, dans lequel le dispositif (10) est agencé pour se fixer au site de traitement.

**4.** Dispositif (10) selon l'une quelconque des revendications précédentes, dans lequel le dispositif (10) est fixé au site de traitement par un adhésif (90) positionné sur la première membrane (40) ou la seconde membrane (60).

**5.** Dispositif (10) selon l'une quelconque des revendications précédentes, dans lequel le dispositif (10) a des bords et dans lequel le dispositif (10) présente une forme formée à partir des bords du dispositif (10), la forme étant rectangulaire, carrée, triangulaire, ovale, trapézoïdale ou circulaire.

**6.** Dispositif (10) selon l'une quelconque des revendications précédentes, dans lequel le corps en polymère microporeux hydrophobe souple (41) de la première membrane (40) présente au moins l'une des caractéristiques suivantes :

il est fait de polytétrafluoroéthylène, et

il est revêtu d'une substance antibactérienne et d'une substance médicale.

7. Dispositif (10) selon l'une quelconque des revendications précédentes, dans lequel le corps en polymère flexible (61) de la seconde membrane (60) présente au moins l'une des caractéristiques suivantes :

il est non poreux, et
il est fait de polytétrafluoroéthylène.

8. Dispositif (10) selon l'une quelconque des revendications précédentes, dans lequel le dispositif (10) comprend en outre au moins l'un des éléments suivants :

une vanne de détente (95), de façon à pouvoir dévier le gaz introduit dans la chambre intérieure (50) du dispositif (10) depuis la chambre intérieure (50) et à travers la vanne de détente (95) pour maintenir une pression de gaz souhaitée dans tout le site de traitement ;
un port de conduite de purge (70), de façon à pouvoir dévier le gaz introduit dans la chambre intérieure (50) du dispositif (10) depuis la chambre intérieure (50) et à travers le port de conduite de purge (70), pour purger le gaz introduit à travers le dispositif (10) ;
un matériau de support d'éponge à alvéoles ouverts (55) positionné dans la chambre intérieure (50) en contact direct avec le corps en polymère microporeux souple de la première membrane (40) ; et
des structures pouvant être mises en forme positionnées dans tout le dispositif (10) et agencées pour conformer le dispositif (10) autour du site de traitement.

9. Dispositif (10) selon l'une quelconque des revendications précédentes, dans lequel le site de traitement est une plaie ou un site chirurgical.

10. Dispositif (10) selon l'une quelconque des revendications précédentes, dans lequel lorsque le corps en polymère microporeux hydrophobe souple de la première membrane (40) présente une dimension des pores de 0,2 μm ou plus, le corps en polymère microporeux hydrophobe souple de la première membrane (40) présente une dimension des pores comprise entre 0,2 μm et 0,4 μm.

11. Système comprenant une source de gaz (20) et un dispositif (10) selon l'une quelconque des revendications 1 à 10.

FIG. 1

FIG. 2

EP 3 928 753 B1

FIG. 3

FIG. 4

FIG. 5

FIG. 6

**EP 3 928 753 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018318566 A